# EUROPEAN PATENT APPLICATION

(11) **EP 4 692 774 A1**
(43) Date of publication of application: **11.02.2026**
(21) Application number: 23934689.3
(22) Date of filing: 17.05.2023
(51) Int. Cl.: G01N 27/26, G01N 27/48

(54) **ELECTROCHEMICAL NUCLEIC ACID DETECTION SENSING ELEMENT AND ELECTROCHEMICAL NUCLEIC ACID DETECTION METHOD BASED ON RPA AND CRISPR/CAS**

(30) Priority: 25.04.2023 CN 202310456634
(71) Applicant: Suzhou Zhong Ke Su Jing Biotechnology Co., Ltd., Suzhou, Jiangsu 215531 (CN); Institute of Process Engineering, Chinese Academy of Sciences, Beijing 100190 (CN)
(72) Inventor: ZHOU, Lei, Suzhou, Jiangsu 215531 (CN); DONG, Jinying, Suzhou, Jiangsu 215531 (CN); WU, Xiaoya, Suzhou, Jiangsu 215531 (CN); SHAO, Gaoxiang, Suzhou, Jiangsu 215531 (CN); ZHANG, Yue, Suzhou, Jiangsu 215531 (CN); MENG, Fanwei, Suzhou, Jiangsu 215531 (CN); DU, Yingxia, Suzhou, Jiangsu 215531 (CN); HU, Qiushi, Suzhou, Jiangsu 215531 (CN); SUN, Chongsi, Suzhou, Jiangsu 215531 (CN)
(74) Representative: Bryn Aarflot AS
(86) International application number: PCT/CN2023/094830
(87) International publication number: WO 2024/221502

(57) **Abstract**

The present application relates to the field of electrochemical analysis and detection, and in particular to an electrochemical nucleic acid detection sensing element and electrochemical nucleic acid detection method based on RPA and CRISPR/Cas. According to the present application, RPA technology, a CRISPR/Cas tool, and electrochemical detection technology are combined, and additionally, an electrochemical active molecule and a modification group are linked to a reporter molecule in a CRISPR/Cas reaction system, such that construction of an immobilization-free and homogeneous electrochemical nucleic acid detection method is realized, and detection of the target nucleic acid can be realized.

## Description

### CROSS REFERENCE TO RELATED APPLICATIONS

The present application claims the benefit of priority to the Chinese Patent Application No. 202310456634.7 filed with China National Intellectual Property Administration on April 25, 2023 and entitled "Electrochemical Nucleic Acid Detection Sensing Element and Electrochemical Nucleic Acid Detection method based on RPA and CRISPR/Cas", which is incorporated herein by reference in its entirety.

### TECHNICAL FIELD

The present disclosure relates to the field of electrochemical analysis and detection, and in particular to an electrochemical nucleic acid detection sensing element and electrochemical nucleic acid detection method based on RPA and CRISPR/Cas.

### BACKGROUND

In recent years, polymerase chain reaction (PCR) technology has been widely used as a primary detection method on a large scale. However, it still suffers from several significant defects, including cumbersome procedures, low portability of instruments, limited detection sensitivity, and a strong dependence on skilled personnel and environmental conditions. The integration of recombinase polymerase amplification (RPA) technology, clustered regularly interspaced short palindromic repeats (CRISPR)-based detection technology, and electrochemical detection technology is expected to establish a foundation for widespread application by non-professional users.

RPA differs from conventional quantitative fluorescence PCR in that it does not require repeated thermal cycling of denaturation-annealing-extension at 56°C, 65°C, and 95°C. Instead, as a commonly used isothermal amplification technique, RPA offers advantages of rapid reaction speed, high detection sensitivity, and low temperature requirements, enabling target nucleic acid amplification at a constant temperature of 37°C.

Since its discovery, the CRISPR/Cas tool has attracted widespread attention as a nucleic acid cleavage tool. With its significant advantages such as high sensitivity, strong specificity, rapid reaction rate, simple operation, and high efficiency, the CRISPR/Cas tool not only plays a unique role in gene editing but also provides new possibilities for nucleic acid detection. The CRISPR/Cas nucleic acid detection tool is formed by a Cas protein, CRISPR RNA (crRNA), and a reporter molecule. The Cas protein and the crRNA are capable of forming a stable binary complex. Depending on the properties of the Cas protein (such as Cas9, Cas12, Cas13, or Cas14), crRNA can selectively bind to RNA or single-stranded DNA (ssDNA) in a sample, forming a ternary complex of Cas/crRNA/RNA or Cas/crRNA/ssDNA. The ternary complex induces conformational changes in the Cas protein that confer trans-cleavage activity toward RNA or ssDNA substrates. The reporter molecule is formed by a DNA or RNA sequence conjugated with a signal molecule. The Cas ternary complex cleaves the DNA or RNA sequence within the reporter molecule, and a free signal molecule is released, thereby generating a detectable optical or colorimetric signal. As a nucleic acid cleavage tool, CRISPR/Cas has been widely used in nucleic acid detection in recent years due to its advantages, including high sensitivity, strong specificity, rapid response, simple operation, and high efficiency.

Combining RPA technology with CRISPR/Cas tool can further enhance detection sensitivity and enable nucleic acid detection at a single molecule level. However, existing nucleic acid detection systems based on RPA-CRISPR/Cas integration mostly utilize fluorescence signals as an output format, which requires expensive supporting instruments and lacks portability.

### SUMMARY

In view of the foregoing deficiencies, the present disclosure aims to provide an electrochemical nucleic acid detection sensing element and electrochemical nucleic acid detection method based on RPA and CRISPR/Cas.

Therefore, the present disclosure implements the following technical solutions:
an electrochemical nucleic acid detection sensing element based on RPA and CRISPR/Cas, including a CRISPR/Cas reaction system and an RPA reaction system;
the CRISPR/Cas reaction system includes an immobilization-free reporter molecule, and the reporter molecule is a non-specific nucleic acid molecule;
the reporter molecule includes:
   at least one electrochemical active molecule; and
   at least one nucleotide.

The reporter molecule is a non-specific nucleic acid molecule that does not participate in pairing recognition.

Optionally, the reporter molecule includes at least one modification group;
the modification group is modified at one end, in a middle, or at both ends of the reporter molecule; and/or
the modification group is a macromolecule having steric hindrance that does not produce an interference peak near a redox peak of an electrochemical active substance.
the CRISPR/Cas reaction system further includes a CRISPR/Cas tool and a reaction buffer.

Optionally, the CRISPR/Cas reaction system further includes a T7 polymerase and rNTP; and/or
the modification group is at least one of carboxyfluorescein, biotin, and digoxigenin.

Optionally, the CRISPR/Cas tool includes any one of CRISPR/Cas9, CRISPR/Cas12, CRISPR/Cas13 and CRISPR/Cas14; and the CRISPR/Cas tool includes a Cas protein and crRNA.

Optionally, a molar concentration ratio of the Cas protein to the crRNA is 4:1-1:4; and/or
a concentration of the reporter molecule is 1-50 µM; and/or
a concentration of the T7 polymerase is 1-10 U/µL; and/or
a concentration of the rNTP is 0.1-2 mM; and/or
a concentration of the reaction buffer is 0.2-1.5×.

Optionally, the molar concentration ratio of the Cas protein to the crRNA is 2:1; and/or
the concentration of the reporter molecule is 5-10 µM; and/or
the concentration of the T7 polymerase is 4-8 U/µL; and/or
the concentration of the rNTP is 0.5-1 mM; and/or
the concentration of the reaction buffer is 0.4-1×.

Optionally, the non-specific nucleic acid molecule is a DNA strand, an RNA strand, or a DNA/RNA hybrid strand, with a length being 5-20 bases; and/or
the electrochemical active molecule is at least one of methylene blue, ferrocene, and thionine; and/or
the electrochemical active molecule is modified at one end, in a middle, or at both ends of the reporter molecule; and/or
the RPA reaction system includes a target-specific amplification primer pair, an enzyme mixture, a reaction buffer, and magnesium acetate; and/or
a concentration of each primer in the amplification primer pair is 150-600 nM; and/or
a concentration of the enzyme mixture is 0.8-1.2×; and/or
a concentration of the reaction buffer is 0.5-1.1×; and/or
a concentration of magnesium acetate is 20-35 mM.

An electrochemical nucleic acid detection method based on RPA and CRISPR/Cas includes:
a method a:
   adding a sample to be detected to a mixed reaction system of a CRISPR/Cas reaction system and an RPA reaction system using the electrochemical nucleic acid detection sensing element based on RPA and CRISPR/Cas for reaction to enable amplification and electrochemical detection; or
a method b:
   adding a sample to be detected to an RPA reaction system first for amplification using the electrochemical nucleic acid detection sensing element based on RPA and CRISPR/Cas, and then adding a resulting reaction product to a CRISPR/Cas reaction system for electrochemical detection.

Optionally, in the method a, a reaction duration is ≥ 10 min, and a reaction temperature is 30-42°C; and/or
in the method b, an amplification reaction duration of the RPA reaction system is ≥ 5 min, and a reaction temperature is 30-42°C; and/or
in the method b, a reaction duration of the CRISPR/Cas reaction system is ≥5 min, and a reaction temperature is 5-50°C; and/or
in the method a or b, a volume of the sample to be detected is 1-10 µL; and/or
in the method b, a volume of the amplification product is 1-50 µL; and/or
in the method a or b, the electrochemical detection is performed using a voltammetry.

Optionally, in the method a, the reaction duration is ≥ 10 min, and the reaction temperature is 35-40°C; and/or
in the method b, the amplification reaction duration of the RPA reaction system is 15-40 min, and the reaction temperature is 35-40°C; and/or
in the method b, the reaction duration of the CRISPR/Cas reaction system is 15-30 min, and the reaction temperature is 20-40°C; and/or
in the method a or b, the volume of the sample to be detected is 3-8 µL; and/or
in the method b, the volume of the amplification product is 5-20 µL; and/or
in the method a or b, the voltammetry includes a cyclic voltammetry, a square wave voltammetry, or a differential pulse voltammetry.

The technical solutions of the present disclosure have the following advantages:
1. The present disclosure provides an electrochemical nucleic acid detection sensing element based on RPA and CRISPR/Cas, including a CRISPR/Cas reaction system and an RPA reaction system; the CRISPR/Cas reaction system includes an immobilization-free reporter molecule, and the reporter molecule is a non-specific nucleic acid molecule; the reporter molecule includes: at least one electrochemical active molecule and at least one nucleotide. During research, the present disclosure discovered that in the electrochemical nucleic acid detection based on CRISPR/Cas, one end of the reporter molecule is generally connected to an electrochemical active molecule, and the other end thereof is immobilized on the electrode surface. As the reaction proceeds, the reporter molecule is cleaved, and the electrochemical active substance is released and moves away from the electrode surface, leading to a decrease in the electrical signal and enabling electrochemical detection. The research concept has been limited to the strategy of "immobilizing on electrode and releasing away from electrode". However, the present disclosure discovered that the strategy of "immobilizing on electrode and releasing away from electrode" results in several drawbacks. Specifically, in terms of "precision and repeatability", since the reporter molecule must be immobilized on the electrode surface, leading to complex electrode fabrication, significant batch-to-batch variation, and the inability to reuse. In terms of "sensitivity and specificity", the solid-liquid reaction mode limits the molecular Brownian motion and collision efficiency, and fails to prevent nonspecific collisions from occurring, resulting in low cleavage efficiency of the reporter molecule during sensitive identification, and generating interference signals by collisions between free active molecules and electrodes during specific detection. In terms of "quantitative capability and detection limit", the inverse correlation between detection signal and target concentration makes it difficult to eliminate high noise in the detection results, resulting in poor linearity of the response, difficulty in extracting weak signals, and an unsatisfactory detection limit, which collectively impose serious constraints on the improvement of detection performance. The present disclosure discovered that when the reporter molecule is immobilization-free and freely dispersed in the reaction system, both the reporter molecule and the CRISPR/Cas ternary complex exist in a homogeneous phase, which significantly improves molecular collision efficiency and consequently increases cleavage efficiency. Furthermore, after the reporter molecule is cleaved, the released molecules of electrochemical active substance are smaller in size and have a higher diffusion rate. This allows them to generate signals upon collision with the electrode more effectively than the reporter molecule, resulting in a clear electrochemical signal intensity that is positively correlated with the target concentration, thereby achieving higher sensitivity and establishing a positive correlation response. In summary, the present disclosure constructs an electrochemical nucleic acid detection sensing element based on RPA and CRISPR/Cas that features fast signal response, simple structure, and enables simple, sensitive, rapid, accurate, and cost-effective detection of target nucleic acid, and has a wide range of applications and high portability.
2. The present disclosure provides an electrochemical nucleic acid detection sensing element based on RPA and CRISPR/Cas, where the reporter molecule includes at least one modification group, the modification group is modified at one end, in a middle, or at both ends of the reporter molecule, and/or the modification group is a macromolecule having steric hindrance that does not produce an interference peak near a redox peak of an electrochemical active substance. During research, the present disclosure discovered that when the modification group is introduced onto the reporter molecule that is linked to the electrochemical active molecule, the steric hindrance of the reporter molecule increases. The greater steric hindrance, along with the wrapping and entanglement of the modification group around nucleic acid molecule, hinders electron transfer of the electrochemical active molecule, thereby effectively eliminating the background signal from free reporter molecules and enabling the extraction of weak signals with low detection limits. Based on the mechanism discovered above, the present disclosure breaks through the conventional design concept of electrochemical sensor. By integrating RPA technology, the CRISPR/Cas tool and electrochemical detection technology, the reporter molecule in the CRISPR/Cas reaction system is linked to both the electrochemical active molecule and the modification group. Before the reporter molecule is cleaved, the presence of the modification group of the reporter molecule leads to extremely weak electrical signals and reduced background signals. After the reporter molecule is cleaved, the electrochemical active molecule is released, exhibits reduced steric hindrance and full dissociation, increased diffusion coefficient and enhanced electrode contact probability, thereby greatly enhancing the electrical signal and producing a strong specific recognition signal.
3. The present disclosure provides an electrochemical nucleic acid detection method based on RPA and CRISPR/Cas, which breaks through the conventional design concept of electrochemical sensor, and integrates RPA technology, the CRISPR/Cas tool and electrochemical detection technology. Additionally, an electrochemical active molecule and a modification group are linked to a reporter molecule in a CRISPR/Cas reaction system, thereby realizing the construction of an immobilization-free and homogeneous electrochemical nucleic acid detection method. The method achieves simple, sensitive, rapid, accurate, and cost-effective detection of target nucleic acid, and has a wide range of applications and high portability.

### BRIEF DESCRIPTION OF THE DRAWINGS

In order to more clearly illustrate technical solutions in the specific implementations of the present disclosure or in the prior art, a brief introduction to the accompanying drawings required for the description of the specific implementations or the prior art will be provided below. Obviously, the accompanying drawings in the following description are some of the implementations of the present disclosure, and those of ordinary skill in the art may still derive other drawings from these accompanying drawings without any creative effort.
FIG. 1 illustrates electrical signal detection results in Test Example 1 using the methods of Examples 1 and 7 according to the present disclosure.
FIG. 2 illustrates electrical signal detection results in Test Example 1 using the methods of Examples 4 and 8 according to the present disclosure.

### DETAILED DESCRIPTIONS OF THE EMBODIMENTS

The following embodiments are provided to facilitate a better understanding of the present disclosure. However, these embodiments are not intended to limit the content and scope of protection of the present disclosure. Any products identical or substantially similar to those of the present disclosure that are derived from the teachings of the present disclosure by combining features of the present disclosure with other prior art techniques by any person should fall within the scope of protection of the present disclosure.

For test steps or conditions not specifically indicated in the embodiments, the conventional test steps or conditions described in the literature of the field can be followed. Reagents or instruments used without indicating manufacturers are conventional products commercially available.

In the examples described below:
a 1× CRISPR/Cas12areaction buffer refers to NEB buffer 3.0 or 3.1;
a 1× CRISPR/Cas13a reaction buffer is formed by 20 mM HEPES-Na (pH 6.8), 50 mM KCl, 10 mM MgCl₂, and 5% glycerol;
a 1× RPA reaction buffer is formed by 50 mM Tris (pH 8.4), 70 mM potassium acetate, 15 mM magnesium acetate, 2 mM DTT, 5% polyethylene glycol, 200 µM dNTP, 3 mM ATP, 20 mM creatine phosphate, and 100 ng/µL creatine kinase; and
a 1× enzyme mixture is formed by 125 ng/µL UvsX recombinase, 600 ng/µL Gp32 single-strand binding protein, 30 ng/■ UvsY recombinase, and 20 ng/µL strand-displacing DNA polymerase.

### Example 1

This example provides an electrochemical nucleic acid detection sensing element based on RPA and CRISPR/Cas13a, including:
an RPA reaction system and a CRISPR/Cas 13a reaction system were constructed as follows:
RPA reaction system: 400 nM of each amplification primer pair, 1× enzyme mixture, 1× RPA reaction buffer, and 28 mM magnesium acetate, with the remaining volume adjusted with water.

CRISPR/Cas13a reaction system: 7 µM reporter molecule, 60 nM Cas13a protein, 30 nM crRNA, 1× CRISPR/Cas13a reaction buffer, 5 U/µL T7 polymerase, and 1 mM rNTP, with the remaining volume adjusted with water.

The reporter molecule is formed by an RNA strand, methylene blue (MB), and biotin. A sequence of the RNA strand is consistent with the cleavage tendency of the Cas13a protein and has a length of 6 bases. A nucleotide sequence of the reporter molecule corresponds to a nucleotide sequence (see the biotin reporter in the table under ssRNA reporter in Table S1 therein) of the biotin reporter molecule in the Cas13a-SHERLOCK (Ding R, Shen Y, Yuan M, Zheng X, Chen S, Duan G. Rapid and facile detection of HBV with CRISPR/Cas13a. New Journal of Chemistry, 2022; 46(41) : 19997-20004), with the biotin modified at a 5' end, and the methylene blue (MB) modified at a 3' end.

A preparation method of the reporter molecule includes the following steps:
(1) a solid-phase CPG support labeled with biotin at a 5' end (commercially available) was loaded into an automated synthesizer (ABI, Model 394), and an oligonucleotide chain was synthesized in a 5'→3' direction. After completion of synthesis, the CPG powder was collected;
(2) 0.5 mL of 25% aqueous ammonia was added to the CPG support, followed by incubation at 55°C for 8 h to allow the oligonucleotide chain to be released from the solid-phase support;
(3) 2 equivalents of 1 mol/L tetrabutylammonium fluoride (TBAF) solution in tetrahydrofuran (THF) were added and incubated at room temperature for 5 h to expose a silanol group at a 2' position;
(4) after impurities were removed, the resulting product and 5 equivalents of 0.5 mol/L methylene blue N-hydroxysuccinimide ester (MB-NHS) in dimethyl sulfoxide (DMSO) solution were added in a sodium bicarbonate solution (pH = 9) for incubation at 50°C for 8 h, to yield a desired sequence; and
(5) a resulting sample was subjected to ultrafiltration, followed by purification using preparative high-performance liquid chromatography (HPLC) to obtain the reporter molecule.

This example further provides an electrochemical nucleic acid detection method using the aforesaid electrochemical nucleic acid detection sensing element based on RPA and CRISPR/Cas13a, including:
S1. 3 µL of a sample to be detected (which may be either an extraction-free product or an extracted and purified nucleic acid) was added to an RPA reaction system (with a volume of 50 µL), and incubated at 37°C for 20 min.
S2. A gold electrode was connected to an electrochemical workstation, and then immersed in a CRISPR/Cas13a reaction system.
S3. 6 µL of an amplified product from the RPA reaction was added to the CRISPR/Casl3a reaction system (with a volume of 30 µL), and incubated at 37°C for 25 min.
S4. After the reaction was completed, an electrochemical signal of methylene blue (MB) in the CRISPR/Cas13a reaction system was detected using a square wave voltammetry.

The detection principle of the method is as follows: When a target DNA is present in the sample, the target DNA can be amplified in large quantities in the RPA reaction system, and the amplification product is transcribed into a target RNA in the CRISPR/Cas13a reaction system with the assistance of T7 polymerase and rNTP. The target RNA forms a ternary complex with the Cas13a protein and crRNA, which activates the trans-cleavage activity of the Casl3a protein, cleaving an RNA strand in the reporter molecule and releasing methylene blue. In contrast, when no target DNA is present in the sample, neither amplification nor transcription is initiated, the trans-cleavage activity of the Casl3a protein remains inactivated, and an RNA strand in the reporter molecule is not cleaved. Consequently, methylene blue modified therein cannot be released. Since a free and intact reporter molecule is modified with a group, it creates significant steric hindrance and is surrounded by nucleic acid and a modification group. The methylene blue modified therein cannot transfer electrons to an electrode. Therefore, when no target DNA is present in the sample, an electrochemical signal of methylene blue in the CRISPR/Cas13a reaction system is extremely weak, resulting in a low background signal. Conversely, the methylene blue itself is small in volume, diffuses rapidly, and exhibits minimal steric hindrance. Therefore, when a target DNA is present in the sample to be detected, an electrochemical signal of methylene blue in the CRISPR/Cas13a reaction system is strong. In summary, the method achieves simple, sensitive, rapid, accurate, and cost-effective detection of target nucleic acid, and has a wide range of applications and high portability.

### Example 2

This example differs from Example 1 in that the CRISPR/Cas13a reaction system is formed by: 1 µM reporter molecule, 2 µM Cas13a protein, 0.5 µM crRNA, 1.5× CRISPR/Casl3a reaction buffer, 10 U/µL T7 polymerase, and 2 mM rNTP, with the remaining volume adjusted with water;
in the step S1, the reaction was carried out at 30°C for 5 min; and
in the step S3, the reaction was carried out at 5°C for 10 min.

### Example 3

This example differs from Example 1 in that the CRISPR/Casl3a reaction system is formed by: 10 µM reporter molecule, 0.5 µM Cas13a protein, 2 µM crRNA, 0.2× CRISPR/Casl3a reaction buffer, 6 U/µL T7 polymerase, and 0.8 mM rNTP, with the remaining volume adjusted with water;
in the step S1, the reaction was carried out at 42°C for 20 min; and
in the step S3, the reaction was carried out at 50°C for 5min.

### Example 4

This example provides an electrochemical nucleic acid detection sensing element based on RPA and CRISPR/Cas12a, including:
a mixed RPA and CRISPR/Cas12a reaction system was constructed as follows:
400 nM of each amplification primer pair, 1× enzyme mixture, 0.6× RPA reaction buffer, 25 mM magnesium acetate, 10 µM reporter molecule, 500 nM Cas12a protein, 250 nM crRNA, and 0.8× CRISPR/Cas12a reaction buffer, with the remaining volume adjusted with water.

The reporter molecule is formed by a DNA strand, methylene blue (MB), and carboxyfluorescein (FAM). A sequence of the DNA strand is consistent with the cleavage tendency of a CRISPR/Cas12a protein and has a length of 10 bases. A nucleotide sequence of the reporter molecule corresponds to a nucleotide sequence (see the surface probe-10nt in the table of the materials therein) of a surface probe in E-CRISPR (Dai Y, Somoza RA, Wang L, Welter JF, Li Y, Caplan AI, et al. Exploring the Trans-Cleavage Activity of CRISPR-Cas12a (cpfl) for the Development of a Universal Electrochemical Biosensor. Angew Chem Int Ed Engl. 2019; 58(48):17399-405), with the FAM modified at a 5' end, and the MB modified at a 3' end.

A preparation method of the reporter molecule includes the following steps:
(1) a solid-phase CPG support labeled with MB at a 3' end was loaded into an automated synthesizer (ABI, Model 394), an oligonucleotide chain was synthesized in a 3'→5' direction, and a desired sequence was obtained by coupling with a commercially available FAM phosphoramidite reagent for incubating at 50°C for 8 h;
(2) a resulting product was released and deprotected using a mixture of tert-butylamine : methanol : water (1:1:2, v/v/v), followed by incubation at 65°C in a water bath for 3 h; and
(3) a resulting sample was subjected to ultrafiltration, followed by purification using preparative HPLC to obtain the reporter molecule.

This example further provides an electrochemical nucleic acid detection method using the aforesaid electrochemical nucleic acid detection sensing element based on RPA and CRISPR/Cas12a, including:
S1. A gold electrode was connected to an electrochemical workstation, and then immersed in an RPA and CRISPR/Cas12a reaction system.
S2. 3 µL of a sample to be detected was added to the RPA and CRISPR reaction system (50 µL), and incubated at 37°C for 25 min. The sample to be detected may be either an extraction-free product or an extracted and purified nucleic acid.
S3. After the reaction was completed, an electrochemical signal of methylene blue in the RPA and CRISPR/Cas12a reaction system was detected using a differential pulse voltammetry.

### Example 5

This example differs from Example 4 in that a 50 µL mixed RPA and CRISPR/Cas12a reaction system was constructed:
150 nM of each amplification primer pair, 0.8× enzyme mixture, 0.8× RPA reaction buffer, 20 mM magnesium acetate, 1 µM reporter molecule, 50 nM Cas12a protein, 50 nM crRNA, and 0.6× CRISPR/Cas12a reaction buffer, with the remaining volume adjusted with water.

In the step S2, the reaction was carried out at 30°C for 30 min.

### Example 6

This example differs from Example 4 in that a 50 µL mixed RPA and CRISPR/Cas12a reaction system was constructed:
600 nM of each amplification primer pair, 1× enzyme mixture, 0.5× RPA reaction buffer, 35 mM magnesium acetate, 15 µM reporter molecule, 1 µM Cas12a protein, 1 µM crRNA, and 1.5× CRISPR/Cas12a reaction buffer, with the remaining volume adjusted with water.

In the step S2, the reaction was carried out at 42°C for 10 min.

### Example 7

This example differs from Example 1 in that the reporter molecule does not contain biotin as a modification group.

In the step (1) of the preparation method of the reporter molecule, a common solid-phase CPG support (that is, a solid-phase CPG support not labeled with biotin at a 5' end) was used.

### Example 8

This example differs from Example 4 in that the reporter molecule does not contain carboxyfluorescein (FAM) as a modification group.

In the step (1) of the preparation method of the reporter molecule, FAM was not added as a synthetic raw material.

### Test Example 1

Sequences of the amplification primer pair and crRNA were derived from RPA-F/R and crRNA (see crRNA 1, F2 and R2 in the RAA primer of crRNA in Table S1 therein) in the literature (Ding R, Shen Y, Yuan M, Zheng X, Chen S, Duan G. Rapid and facile detection of HBV with CRISPR/Cas13a. New Journal of Chemistry. 2022; 46(41) : 19997-20004.) Subsequent operation was implemented according to Example 1 and Example 7, as described below:
S1. An RPA reaction system was constructed as follows:
400 nM of each amplification primer pair, 1× enzyme mixture, 1× RPA reaction buffer, and 28 mM magnesium acetate, with the remaining volume adjusted with water.

S2. A CRISPR/Cas13a reaction system was constructed as follows:
7 µM reporter molecule, 60 nM Cas13a protein, 30 nM crRNA, 1× CRISPR/Casl3a reaction buffer, 5 U/µL T7 polymerase, and 1 mM rNTP, with the remaining volume adjusted with water.

S3. 3 µL of the extracted and purified nucleic acid (with a concentration of approximately 10 copies/µL) was added to an RPA reaction system (with a volume of 50 µL), and incubated at 37°C for 20 min.

S4. A gold electrode was connected to an electrochemical workstation, and then immersed in a CRISPR/Cas13a reaction system.

S5. 6 µL of an amplified product from the RPA reaction was added to the CRISPR/Casl3a reaction system (with a volume of 30 µL), and incubated at 37°C for 25 min.

S6. After the reaction was completed, an electrochemical signal of methylene blue (MB) in the CRISPR/Cas13a reaction system was detected using a square wave voltammetry.

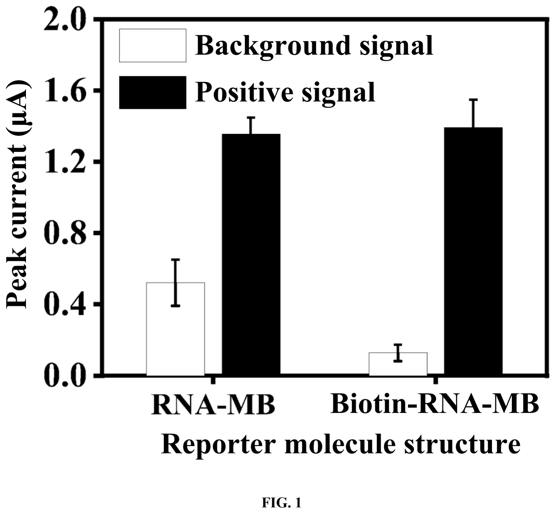

Results are shown in FIG. 1. The biotin-RNA-MB in the figure is the reporter molecule in Example 1, and the RNA-MB is the reporter molecule in Example 7. As can be seen from the figure, the reporter molecules in Examples 1 and 7 both produced strong positive signals and weak background signals. Further comparison between Example 1 and Example 7 revealed that Example 1 exhibited a lower background signal and a higher positive signal, indicating that the biotin-RNA-MB reporter molecule in Example 1 provided a lower background signal and a better signal-to-noise ratio.

### Test Example 2

Sequences of the amplification primer pair and crRNA were derived from the Bl FL-RPA and crRNA (see crRNA, and primers B1-RPA-F/B1-RPA-R in Table S1 therein) in the literature (Lei R, Li L, Wu P, Fei X, Zhang Y, Wang J, et al. RPA/CRISPR/Cas12a-Based On-Site and Rapid Nucleic Acid Detection of Toxoplasma gondii in the Environment. Acs Synth Biol. 2022; 11 (5) : 1772-81). Subsequent operation was implemented according to Example 4 and Example 8, as described below:
S1. A mixed system of the RPA and CRISPR/Cas12a reaction system was constructed as follows:
400 nM of each amplification primer pair, 1× enzyme mixture, 0.6× RPA reaction buffer, 25 mM magnesium acetate, 10 µM reporter molecule, 500 nM Cas12a protein, 250 nM crRNA, and 0.8× CRISPR/Cas12a reaction buffer, with the remaining volume adjusted with water.

S2. A gold electrode was connected to an electrochemical workstation, and then immersed in an RPA and CRISPR/Cas12a reaction system.

S3. 3 µL of the extracted and purified nucleic acid (with a concentration of approximately 10 copies/µL) was added to the RPA and CRISPR reaction system (50 µL), and incubated at 37°C for 25 min.

S4. After the reaction was completed, an electrochemical signal of methylene blue in the RPA and CRISPR/Cas12a reaction system was detected using a differential pulse voltammetry.

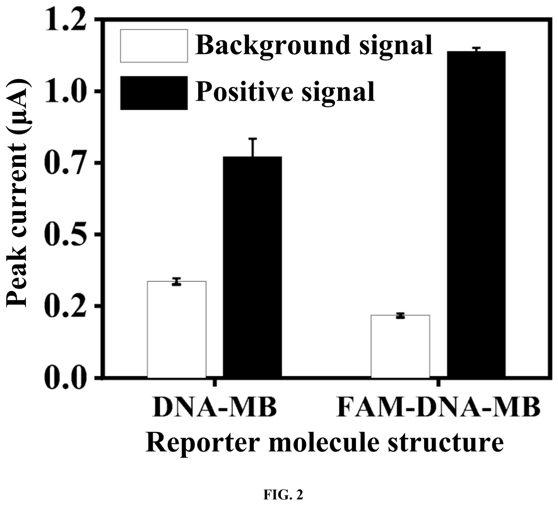

Results are shown in FIG. 2. The FAM-DNA-MB in the figure is the reporter molecule in Example 4, and the DNA-MB is the reporter molecule in Example 8. As can be seen from the figure, the reporter molecules in Example 4 and Example 8 both produced strong positive signals and weak background signals. Further comparison between Example 4 and Example 8 revealed that Example 4 exhibited a lower background signal and a higher positive signal, indicating that the FAM-DNA-MB reporter molecule in Example 4 provided a lower background signal and a better signal-to-noise ratio.

Apparently, the above embodiments are merely examples given for clearly illustrating the present disclosure, and are not intended to limit the implementations. For those of ordinary skill in the art, modifications or variations in other forms may be made on the basis of the above description. It is unnecessary to exhaust all implementation modes herein. Obvious modifications or variations made to the present disclosure shall still fall within the scope of protection of the present disclosure.

## Claims

1. An electrochemical nucleic acid detection sensing element based on RPA and CRISPR/Cas, comprising a CRISPR/Cas reaction system and an RPA reaction system; wherein
the CRISPR/Cas reaction system comprises an immobilization-free reporter molecule, and the reporter molecule is a non-specific nucleic acid molecule; wherein
the reporter molecule contains:
at least one electrochemical active molecule; and
at least one nucleotide.

2. The electrochemical nucleic acid detection sensing element based on RPA and CRISPR/Cas according to claim 1, wherein the reporter molecule contains at least one modification group:
the modification group is modified at one end, in a middle, or at both ends of the reporter molecule; and/or
the modification group is a macromolecule having steric hindrance that does not produce an interference peak near a redox peak of an electrochemical active substance; and/or
the CRISPR/Cas reaction system further comprises a CRISPR/Cas tool and a reaction buffer.

3. The electrochemical nucleic acid detection sensing element based on RPA and CRISPR/Cas according to claim 2, wherein the CRISPR/Cas reaction system further comprises a T7 polymerase and rNTP; and/or
the modification group is at least one of carboxyfluorescein, biotin, and digoxigenin.

4. The electrochemical nucleic acid detection sensing element based on RPA and CRISPR/Cas according to claim 3, wherein the CRISPR/Cas tool comprises any one of CRISPR/Cas9, CRISPR/Cas12, CRISPR/Casl3 and CRISPR/Cas14; and the CRISPR/Cas tool comprises a Cas protein and crRNA.

5. The electrochemical nucleic acid detection sensing element based on RPA and CRISPR/Cas according to claim 4, wherein a molar concentration ratio of the Cas protein to the crRNA is 4:1-1:4; and/or
a concentration of the reporter molecule is 1-50 µM; and/or
a concentration of the T7 polymerase is 1-10 U/µL; and/or
a concentration of the rNTP is 0.1-2 mM; and/or
a concentration of the reaction buffer is 0.2-1.5×.

6. The electrochemical nucleic acid detection sensing element based on RPA and CRISPR/Cas according to claim 5, wherein
the molar concentration ratio of the Cas protein to the crRNA is 2:1; and/or
the concentration of the reporter molecule is 5-10 µM; and/or
the concentration of the T7 polymerase is 4-8 U/µL; and/or
the concentration of the rNTP is 0.5-1 mM; and/or
the concentration of the reaction buffer is 0.4-1 ×.

7. The electrochemical nucleic acid detection sensing element based on RPA and CRISPR/Cas according to any one of claims 1-6, wherein the non-specific nucleic acid molecule is a DNA strand, an RNA strand, or a DNA/RNA hybrid strand, with a length being 5-20 bases; and/or
the electrochemical active molecule is at least one of methylene blue, ferrocene, and thionine; and/or
the electrochemical active molecule is modified at one end, in a middle, or at both ends of the reporter molecule; and/or
the RPA reaction system comprises a target-specific amplification primer pair, an enzyme mixture, a reaction buffer, and magnesium acetate; and/or
a concentration of each primer in the amplification primer pair is 150-600 nM; and/or
a concentration of the enzyme mixture is 0.8-1.2×; and/or
a concentration of the reaction buffer is 0.5-1.1×; and/or
a concentration of magnesium acetate is 20-35 mM.

8. An electrochemical nucleic acid detection method based on RPA and CRISPR/Cas, comprising:
a method a:
adding a sample to be detected to a mixed reaction system of a CRISPR/Cas reaction system and an RPA reaction system using the electrochemical nucleic acid detection sensing element based on RPA and CRISPR/Cas according to any one of claims 1-7 for reaction to enable amplification and electrochemical detection; or
a method b:
adding a sample to be detected to an RPA reaction system first for amplification using the electrochemical nucleic acid detection sensing element based on RPA and CRISPR/Cas according to any one of claims 1-7, and then adding a resulting reaction product to a CRISPR/Cas reaction system for electrochemical detection.

9. The electrochemical nucleic acid detection method based on RPA and CRISPR/Cas according to claim 8, wherein
in the method a, a reaction duration is ≥ 10 min, and a reaction temperature is 30-42°C; and/or
in the method b, an amplification reaction duration of the RPA reaction system is ≥ 5 min, and a reaction temperature is 30-42°C; and/or
in the method b, a reaction duration of the CRISPR/Cas reaction system is ≥5 min, and a reaction temperature is 5- 50°C; and/or
in the method a or b, a volume of the sample to be detected is 1-10 µL; and/or
in the method b, a volume of the amplification product is 1-50 µL; and/or
in the method a or b, the electrochemical detection is performed using a voltammetry.

10. The electrochemical nucleic acid detection method based on RPA and CRISPR/Cas according to claim 9, wherein
in the method a, the reaction duration is ≥ 10 min, and the reaction temperature is 35-40°C; and/or
in the method b, the amplification reaction duration of the RPA reaction system is 15-40 min, and the reaction temperature is 35-40°C; and/or
in the method b, the reaction duration of the CRISPR/Cas reaction system is 15-30 min, and the reaction temperature is 20-40°C; and/or
in the method a or b, the volume of the sample to be detected is 3-8 µL; and/or
in the method b, the volume of the amplification product is 5-20 µL; and/or
in the method a or b, the voltammetry comprises a cyclic voltammetry, a square wave voltammetry, or a differential pulse voltammetry.
